# EUROPEAN PATENT SPECIFICATION

(11) **EP 3 586 820 B1**
(45) Date of publication and mention of the grant of the patent: **24.04.2024**
(21) Application number: 18197752.1
(22) Date of filing: 28.09.2018
(51) Int. Cl.: A61Q 13/00, A61K 8/86, A61K 8/39

(54) **METHOD FOR MANUFACTURING AN ALCOHOL-FREE PERFUME**
PROCÉDÉ DE FABRICATION D'UN PARFUM SANS ALCOOL
VERFAHREN ZUR HERSTELLUNG EINES ALKOHOLFREIEN PARFÜMS

(30) Priority: 27.06.2018 PL 42610418
(43) Date of publication of application: 01.01.2020
(73) Proprietor: Finea sp. z o.o., 01-350 Warszawa (PL); Politechnika Krakowska im. Tadeusza Kosciuszki, 31-115 Kraków (PL)
(72) Inventor: Gut, Katarzyna, 01-350 Warszawa (PL); Wolinska-Kennard, Katarzyna, Rochester ME1 3 XS (GB); Sikora, Elzbieta, 31-988 Kraków (PL); Miastkowska, Malgorzata, 30-438 Kraków (PL); Lason , Elwira, 33-332 Kraków (PL)
(74) Representative: Doskoczynska-Groyecka, Anna

(56) References cited:
- EP-A1- 0 516 508
- EP-A1- 0 572 080
- EP-B1- 0 572 080
- WO-A1-2005/123028
- WO-A1-2011/077062
- FR-A1- 2 787 348
- FR-B1- 2 787 348

## Description

The subject of the invention is a method of producing non-alcoholic perfumes with nanoemulsion properties.

In the composition of classic perfumery products on the market, ethanol is used as a solvent for hydrophobic fragrances. Introducing lipophilic systems into water without solubilizer (e.g. ethanol) is extremely difficult. Obtaining stable oil-in-water nanoemulsions containing fragrance compositions, without the addition of ethanol or other solvents (co-surfactants) from the group of polyhydric alcohols such as glycols or glycerin is a technological challenge.

Alcohol-based perfumes present on the market are a standard solution, however, ethyl alcohol is a solvent with a specific irritating potential. Perfumery products in the form of solid and perfumed oils, have a number of disadvantages from the utilitarian point of view, namely, it is not possible to disperse the product on the clothes, in the air, they leave greasy and slippery spots on the perfumed surfaces. Discussed perfumery products have their advantages and disadvantages and therefore new solutions are sought for the physico-chemical form of perfumery preparations. Additionally, to expand the market with new customer groups: adolescents, allergy sufferers, Middle East markets, elimination of the most popular solvent of fragrance compositions, i.e. ethyl alcohol, becomes not only a necessity but also a challenge.

Nanoemulsions are a modern form of cosmetic preparations that is an achievement of nanotechnology. From a physico-chemical point of view, these are liquid colloidal systems characterized by a high degree of dispersion in the range of 20 to 500 nm, consisting of an aqueous phase, an oil phase and a surfactant, sometimes with the addition of a co-surfactant. These systems are characterized by high kinetic stability. Small size of the nanoemulsion particles reduces the effect of the force of gravity, which is overcome by Brownian motion, which prevents the process of sedimentation and creaming. However, from a thermodynamic point of view, nanoemulsions are non-equilibrium systems, which can lead to their destabilization, i.e. flocculation, coalescence and/or Ostwald ripening. In contrast to microemulsions, obtaining them requires the use of smaller amounts of surfactant, which allows to obtain systems that are more safe for the body. Clear appearance, smoothness and low viscosity make nanoemulsions an attractive form for many industrial applications, including cosmetics technology.

In the nanoemulsions described in the literature and obtained on a large scale a single lipophilic component (insoluble in water) is most often used as the oil phase. It should be emphasized that fragrance compositions contain from several to several dozens of compounds with different chemical structures (alcohols, phenols, aldehydes, esters, saturated, unsaturated, cyclic, branched hydrocarbons), making them a difficult base for obtaining stable nanoemulsion systems. Obtaining a water-based perfumes is an extremely difficult task, due to the hydrophobic nature of the fragrance composition and its complex composition.

The main technological problem is to obtain a stable product constituting a mixture of insoluble liquids: water and a hydrophobe fragrance composition in an amount of 3 to 15%, corresponding to the content of the fragrance composition in conventional perfumery products. The nanoemulsion not only acts as a carrier for the fragrance composition but also increases the chemical stability of the compounds in the composition.

The literature describes examples of perfumery products developed on the basis of a safe solvent, i.e. water, so-called "alcohol free". The fragrance compositions used as an essence are by nature a mixture of hydrophobic (water-insoluble) compounds, therefore obtaining stable formulations is very difficult. One of the solutions facilitating the introduction of fragrance compositions into the water is the use of solubilizers, i.e. substances increasing solubility, aroma oils, for example glycols or glycerine, or the use of surface-active compounds of so-called surfactants so as to obtain stable products containing hydrophobic fragrances in the form of emulsions, microemulsions, micelles or liposomes.

Patent literature describes solutions for non-alcoholic perfumes.

Patent application CN103637942 (A) "Alcohol-free transparent perfume composition" discloses a composition with a high concentration of surfactants of more than 10% and the presence of polyhydric alcohols, such as 1,3-butanediol and glycerine.

Patent application US5468725 (A) "Alcohol free perfume" discloses perfumes in the form of a microemulsion containing a high concentration of surfactants, including cationic ones, which irritate the skin.

Patent application US5736505 (A) "Non-alcoholic perfume or cologne" discloses a perfume composition containing an additional solubilizer - glycereth-7-triacetate, at a concentration of from 3 to 12%.

While, EP2127632 A1 "Perfume composition with reduced alcohol content" discloses the composition of glycerine-containing perfumes, an additional hydrophobic solvent, for example, isohexadecane and isoeicosan, and anionic emulsifiers, for example Disodium Lauryl Sulfosuccinate accompanying non-ionic emulsifiers. Also cited are patent documents WO 2011/077062 and EP 572080.

The perfume formulations described in the above patents include, in addition to additional solubilizers, such as polyhydroxyl alcohols or paraffinic hydrocarbons, cationic or anionic surfactants that have the potential to irritate the skin.

The essence of the solution according to the invention consists in that in the first place, in order to obtain a pre-emulsion, a certain amount of oil phase consisting of a fragrance composition and emulsifier and an aqueous phase, being a mixture of water and preservative, is dispersed using a mechanical stirrer with a rotational speed of agitators from 450 to 550 rpm for 10 to 15 minutes. The process is carried out at a temperature of 20°C to 30°C, and the obtained pre-emulsion is then subjected to a homogenization process using an ultrasonic probe with a sonication power of 10 to 30 W. The time for the homogenization process is determined by obtaining a transparent system. The oil phase is obtained by combining from 5 to 10% by weight of an emulsifier and 5 to 15% by weight of a fragrance composition, the weight ratio S: O of the emulsifier (S) to the fragrance composition (O) ranging from 1: 1 to 1 : 3. On the other hand, the aqueous phase is obtained by combining the preservative in an amount of 0.00025 to 1% by weight with water in an amount to make up to 100% by weight of the ingredients.

Preferably, the pre-emulsion process is carried out at 25°C.

Preferably, the sonication power is 15 W.

Preferably, the fragrance composition is a mixture of lipophilic, volatile fragrance compounds with a cyclic, aliphatic or aromatic structure.

Preferably the emulsifier is polyethylene glycol-35 castor oil.

Preferably, the emulsifier is a mixture of polyglycerol-4 esters of sebacic and lauryl acids as well as polyglycerol-6 esters of capric and caprylic acids. Preferably, a preservative is a microbiological purity stabilizer from the group of nAg silver colloids.

Preferably, a preservative is a microbiological purity stabilizer from the group of nAu gold colloids.

Preferably, the preservative is anisic acid or salts thereof.

Preferably, the preservative is levulinic acid or salts thereof.

An advantage of the solution according to the invention is to obtain nanoemulsion formulations compatible with fragrance compositions, without the addition of ethanol or other solvents (co-surfactants) from the polyhydric alcohol group. It is also worth emphasizing that it is not necessary to use an additional solubilizer, for example in the form of a low polarity oil (e.g. isohexadecane), increasing the stability of the system. As emulsifiers, only non-ionic, skin-friendly emulsifiers are used at concentrations that are safe for the body, not exceeding 10%. It should also be emphasized that the pre-emulsification process is preferably carried out at a temperature of 25°C, which significantly reduces the costs of the production process and increases the chemical stability of substances contained in fragrance compositions, sensitive to elevated temperature.

The development of perfumes in the form of a water-based nanoemulsion, eliminates ethanol or other solvents for water. The elimination of alcohol - flammable, and in higher concentrations, irritant solvent - allows to create a safe product for a wider group of consumers. In addition, it is necessary to emphasize the economic aspect related to the international exchange of goods and the reduction of transport costs.

Low viscosity, in the range of from 60 to 110 mPas, facilitates additional application, while the fluid nature of the system gives the nanoemulsion pleasant usable properties.

The object of the inventions is presented in the embodiments.

### Example 1

The components of the oil phase (fragrance composition No. 1) are weighed in an amount of 6% by weight, surfactant (PEG-35 Castor oil) in an amount of 10% by weight, silver colloid (nAg) in an amount of 0.00025% by weight and water in addition to 100% by weight. To obtain a pre-emulsion, a certain amount of the oil phase and the aqueous phase are dispersed by means of a mechanical stirrer in 10 min using a stirrer speed of v = 500 rpm. The process is carried out at 25°C. The obtained pre-emulsion is then subjected to a homogenization process by means of an ultrasonic probe with a 15 W sonication power. The time of the homogenization process is determined by obtaining a transparent system.

The appropriately selected weight ratio of the surfactant to the oil phase ensures a stable nanoemulsion.

A nanoemulsion is obtained with the following composition in % by weight:
- nAg - 0.00025%,
- fragrance composition no. 1 - 6%
- PEG-35 Castor oil - 10%
- water - up to 100%.

### Example 2

The components of the oil phase (fragrance composition No. 2) are weighed in an amount of 6% by weight, surfactant (PEG-35 Castor oil) in an amount of 6% by weight, silver colloid in an amount of 0.00025% by weight and water in addition to 100% by weight. The nanoemulsion procedure described in Example 1 is followed and a nanoemulsion is obtained with the following composition in % by weight:
- nAg - 0.00025%,
- fragrance composition no. 2 - 6%
- PEG-35 Castor oil - 6%
- water - up to 100%.

### Example 3

The components of the oil phase (fragrance composition No. 3) are weighed in an amount of 15% by weight, surfactant (PEG-35 Castor oil) in an amount of 10% by weight, silver colloid (nAg) in an amount of 0.00025% by weight and water in addition to 100% by weight. The nanoemulsion procedure described in Example 1 is followed and a nanoemulsion is obtained with the following composition in % by weight:
- nAu - 0.00025%
- fragrance composition no. (3) - 15%
- PEG-35 Castor oil - 10%
- water - up to 100%.

### Example 4

The components of the oil phase (fragrance composition 4) are weighed in an amount of 6% by weight, surfactant (decyl polyglucoside) in an amount of 10% by weight, silver colloid (nAg) in an amount of 0.00025% and water in addition to 100% by weight. The nanoemulsion procedure described in Example 1 is followed and a nanoemulsion is obtained with the following composition in % by weight:
- nAu - 0.00025%,
- fragrance composition no. (4) - 6%
- decyl glucoside - 10%
- water - up to 100%.

### Example 5

The components of the oil phase (fragrance composition No. 1) are weighed in an amount of 6% by weight, surfactant (a mixture of polyglycerol-4 esters and sebacic and lauryl acids as well as polyglycerol-6 esters and capric and caprylic acids) in an amount of 10% by weight, a mixture of sodium salts of levulinic and anisic acid in an amount of 1% by weight and water in addition to 100% by weight.

To obtain a pre-emulsion, a certain amount of the oil phase and the aqueous phase are dispersed by means of a mechanical stirrer in 12 minutes using a stirrer speed of v = 520 rpm. The process is carried out at constant temperature of 25°C. The obtained pre-emulsion is then subjected to a homogenization process by means of an ultrasonic probe with a 20 W sonication power. The time of the homogenization process is determined by obtaining a transparent system.

A nanoemulsion is obtained with the following composition in % by weight:
- a mixture of sodium salts of levulinic and anisic acids - 1 %
- fragrance composition no. (1) - 6%
- polyglycerol-4 esters and sebacic and lauryl acids as well as polyglycerol-6 esters and capric and caprylic acids - 10%
- water - up to 100%.

The nanoemulsions obtained in Examples 1 to 5 were characterized by average internal particle size of from 20 to 100 nm, transparent appearance, low viscosity between 60 - 100 mPas, (T = 25°C, v = 50 s-1), low surface tension: 28 - 30 mN/m, (T= 25°C), pH corresponding to the physiological pH of the skin.

## Claims

1. A method of producing non-alcoholic perfumes **characterized in that** in the first place, in order to obtain a pre-emulsion, a certain amount of oil phase consisting of a fragrance composition and emulsifier and an aqueous phase, being a mixture of water and preservative, is dispersed using a mechanical stirrer with a rotational speed of agitators from 450 to 550 rpm for 10 to 15 minutes, wherein the process is carried out at a temperature of 20°C to 30°C, and the obtained pre-emulsion is then subjected to a homogenization process using an ultrasonic probe with a sonication power of 10 to 30 W, wherein the time for the homogenization process is determined by obtaining a transparent system and the oil phase is obtained by combining from 5 to 10% by weight of an emulsifier and 5 to 15% by weight of a fragrance composition, the weight ratio S: O of the emulsifier (S) to the fragrance composition (O) ranging from 1: 1 to 1 : 3, on the other hand, the aqueous phase is obtained by combining the preservative in an amount of 0.00025 to 1% by weight with water in an amount to make up to 100% by weight of the ingredients.

2. A method according to claim 1, **characterized in that** the pre-emulsion process is carried out at 25°C.

3. A method according to claim 1, **characterized in that** the sonication power is 15 W.

4. A method according to claim 1, **characterized in that** the fragrance composition is a mixture of lipophilic, volatile fragrance compounds with a cyclic, aliphatic or aromatic structure.

5. A method according to claim 1, **characterized in that** the emulsifier is polyethylene glycol-35 castor oil.

6. A method according to claim 1, **characterized in that** the emulsifier is a mixture of polyglycerol-4 esters of sebacic and lauryl acids as well as polyglycerol-6 esters of capric and caprylic acids.

7. A method according to claim 1, **characterized in that** a preservative is a microbiological purity stabilizer from the group of nAg silver colloids.

8. A method according to claim 1, **characterized in that** a preservative is a microbiological purity stabilizer from the group of nAu gold colloids.

9. A method according to claim 1, **characterized in that** a preservative is anisic acid or salts thereof.

10. A method according to claim 1, **characterized in that** a preservative is levulinic acid or salts thereof.

## Patentansprüche

1. Verfahren zur Herstellung eines alkoholfreien Parfüms, **dadurch gekennzeichnet, dass** man zunächst zur Herstellung einer Voremulsion eine bestimmte Menge der Ölphase, bestehend aus einer Duftstoffzusammensetzung und einem Emulgator sowie einer ein Gemisch aus Wasser und einem Konservierungsmittel bildenden wässrigen Phase mittels eines mechanischen Rührers mit einer Rührgeschwindigkeit von 450 bis 550 U/min für 10 bis 15 Minuten dispergiert, wobei das Verfahren bei einer Temperatur von 20 °C bis 30 °C durchgeführt wird, und die hergestellte Voremulsion anschließend einem Homogenisierungsverfahren unter Verwendung eines Ultraschallgeräts mit einer Sonde mit einer Überschallleistung von 10 bis 30 W unterzogen wird, wobei die Dauer des Homogenisierungsverfahrens so bestimmt wird, dass ein transparentes System und die Ölphase durch Kombination von 5 bis 10 Gew.-% des Emulgators und 5 bis 15 Gew.-% der Duftstoffzusammensetzung hergestellt werden, wobei das Gewichtsverhältnis S : O des Emulgators (S) zur Duftstoffzusammensetzung (O) im Bereich von 1: 1 bis 1 : 3 liegt, während die wässrige Phase durch Kombination von Konservierungsmittel in einer Menge von 0,00025 bis 1 Gew.-% und Wasser in einer bis zu 100 Masse-% der Inhaltsstoffe ergänzenden Menge hergestellt wird.

2. Verfahren nach Anspruch 1, **dadurch gekennzeichnet, dass** das Verfahren zur Herstellung der Voremulsion bei einer Temperatur von 25 °C durchgeführt wird.

3. Verfahren nach Anspruch 1, **dadurch gekennzeichnet, dass** die Überschallleistung 15 W beträgt.

4. Verfahren nach Anspruch 1, **dadurch gekennzeichnet, dass** die Duftstoffzusammensetzung ein Gemisch aus lipophilen, flüchtigen Duftstoffverbindungen mit zyklischer, aliphatischer oder aromatischer Struktur ist.

5. Verfahren nach Anspruch 1, **dadurch gekennzeichnet, dass** der Emulgator das mit 35 Mol Ethylenoxid ethoxylierte Rizinusöl ist.

6. Verfahren nach Anspruch 1, **dadurch gekennzeichnet, dass** der Emulgator eine Mischung aus Polyglycerin-4-Estern und Sebacin- und Laurinsäure sowie Polyglycerin-6-Estern und Caprin- und Caprylsäure ist.

7. Verfahren nach Anspruch 1, **dadurch gekennzeichnet, dass** das Konservierungsmittel ein mikrobiologischer Reinheitsstabilisator aus der Gruppe der nAg-Silberkolloide ist.

8. Verfahren nach Anspruch 1, **dadurch gekennzeichnet, dass** das Konservierungsmittel ein mikrobiologischer Reinheitsstabilisator aus der Gruppe der nAu-Goldkolloide ist.

9. Verfahren nach Anspruch 1, **dadurch gekennzeichnet, dass** das Konservierungsmittel Anissäure oder deren Salze ist.

10. Verfahren nach Anspruch 1, **dadurch gekennzeichnet, dass** das Konservierungsmittel Lävulinsäure oder deren Salze ist.

## Revendications

1. Le processus de fabrication de parfums sans alcool se **caractérise par** plusieurs étapes. Tout d'abord, pour d'obtenir une pré-émulsion, une quantité spécifique de la phase huileuse, constituée de la composition parfumée et de l'émulsifiant, ainsi que de la phase aqueuse, composée d'un mélange d'eau et de conservateur, est dispersée à l'aide d'un mélangeur mécanique à une vitesse du mélangeur de 450 à 550 tr/min pendant 10 à 15 min, et la température du processus est maintenue entre 20°C à 30°C. Ensuite, la pré-émulsion obtenue est soumise à un processus d'homogénéisation à l'aide d'un ultrasoniseur équipé d'une sonde, utilisant une puissance de sonication de 10 à 30 W. La durée du processus d'homogénéisation est déterminée en observant la formation d'une substance transparente. La phase huileuse est obtenue en mélangeant 5 à 10 % en masse d'émulsifiant et 5 à 15 % en masse de la composition parfumée. Le rapport pondéral 5 : O de l'émulsifiant (S) à la composition parfumée (O) varie de 1: 1 à 1 : 3. Quant à la phase aqueuse, elle est obtenue en combinant un conservateur dans une quantité de 0,00025 à 1 % en masse avec de l'eau, qui complète jusqu'à 100 % des ingrédients en masse.

2. Procédé selon la revendication 1, **caractérisé par le fait que** le procédé d'obtention de la pré-émulsion est réalisé à une température de 25 °C.

3. Procédé selon la revendication 1, **caractérisé par le fait que** la puissance de sonication est de 15 W.

4. Procédé selon la revendication 1, **caractérisé par le fait que** la composition parfumée est un mélange de composés odorants lipophiles, volatils, à structure cyclique, aliphatique ou aromatique.

5. Procédé selon la revendication 1, **caractérisé par le fait que** l'émulsifiant est de l'huile de ricin éthoxylée avec 35 moles d'oxyde d'éthylène.

6. Procédé selon la revendication 1, **caractérisé par le fait que** l'émulsifiant est un mélange d'esters de polyglycérol-4 et d'acides sébacique et laurique, ainsi que d'esters de polyglycérol-6 et d'acides caprique et caprylique.

7. Procédé selon la revendication 1, **caractérisé par le fait que** la substance conservatrice est un stabilisant de pureté microbiologique du groupe des colloïdes d'argent nAg.

8. Procédé selon la revendication 1, **caractérisé par le fait que** la substance conservatrice est un stabilisant de pureté microbiologique du groupe des colloïdes d'or nAu.

9. Procédé selon la revendication 1, **caractérisé par le fait que** le conservateur est l'acide anisique ou ses sels.

10. Procédé selon la revendication 1, **caractérisé par le fait que** le conservateur est l'acide lévulinique ou ses sels.
